(19) Europäisches Patentamt

European Patent Office

Office européen des brevets

(11) Veröffentlichungsnummer: **0 445 359 A1**

# (12) EUROPÄISCHE PATENTANMELDUNG

(21) Anmeldenummer: **90119844.0**

(22) Anmeldetag: **16.10.90**

(51) Int. Cl.⁵: **A61N 1/36**

(30) Priorität: **09.03.90 DE 4007542**

(43) Veröffentlichungstag der Anmeldung:
**11.09.91 Patentblatt 91/37**

(84) Benannte Vertragsstaaten:
**AT BE CH DE DK ES FR GB GR IT LI LU NL SE**

(71) Anmelder: **PIERENKEMPER GMBH**
**Gehrnstrasse 5**
**W-6332 Ehringshausen(DE)**

(72) Erfinder: **Kreutner, Bernd**
**Gehrnstrasse 5**
**W-6332 Ehringshausen(DE)**

(74) Vertreter: **Linser, Heinz et al**
**Patentanwälte Heinz Linser Dipl. Ing.**
**Eckhardt Eyer Robert-Bosch-Strasse 12a**
**Postfach 10 22 10**
**W-6072 Dreieich(DE)**

(54) **Vorrichtung zur transkutanen elektrischen Nervenstimulation.**

㊗ Die Erfindung betrifft eine Vorrichtung zur transkutanen elektrischen Nervenstimulation (TNS) mit einer Energieversorgung, einem Frequenzgenerator und einer elektrischen Schaltung zur Erzeugung unterschiedlicher Ausgangsfrequenzen veränderbarer Intensitäten an den mit der Vorrichtung verbindbaren Ausgangselektroden und eine Ausgangskurzschlußschaltung zur Verbesserung der abgegebenen Impulsform. Die Ausgangskurzschlußschaltung weist einen Transistor T1 auf, der bei Beaufschlagung durch einen Ansteuerimpuls mit seinem Kollektor die Basis eines nachgeschalteten Transistors T2 ansteuert, derart, daß der Ansteuerimpuls im Transistor T1 invertiert wird, wobei der Transistor T2 nach der negativen Impulsflanke durchschaltet und der Ausgang über den emitterseitig verbundenen Entladewiderstand R(ent) kurzgeschlossen wird.

FIG. 6

EP 0 445 359 A1

Die Erfindung betrifft eine Vorrichtung zur transkutanen elektrischen Nervenstimulation (TNS) mit einer Energieversorgung, einem Frequenzgenerator und einer elektrischen Schaltung zur Erzeugung unterschiedlicher Ausgangsfrequenzen veränderbarer Intensitäten an den mit der Vorrichtung verbindbaren Ausgangselektroden und eine Ausgangskurzschlußschaltung zur Verbesserung der abgegebenen Impulsform.

Die Behandlung von Schmerzzuständen der unterschiedlichsten Art mit Hilfe elektrischer Nervenstimulation durch stationäre Geräte ist seit langem bekannt und gehört zum Standard der physikalischen Therapie. Hierbei ist die Hautbelastung durch Gleichstrom- oder Stromformen mit Gleichstromcharakter am größten, während die Stromeindringtiefe, welche für die Behandlung vieler Schmerzzustände von Bedeutung ist, bei solchen Stromformen äußerst gering ist.

Erst durch die Entwicklung elektronischer Bauelemente, insbesondere Halbleiter, wurde es möglich Pulsströme und Frequenzen der unterschliedlichsten Art zu erzeugen und deren therapeutische Wirkung zu ermitteln.

Durch die technische Entwicklung im Bereich der Energiequellen und der Miniaturisierung in der Elektronik konnten kleine, tragbare und für einen ausreichenden Dauerbetrieb geeignete Therapiegeräte entwickelt werden, wobei der entscheidende Unterschied zwischen den für stationären Betrieb ausgelegten Hochvolt- und den tragbaren TNS-Geräten lediglich in der unterschiedlichen Leistungsabgabe liegt. Eine wirksame und ausreichende Stromdichte kann bei TNS-Geräten jedoch durch relativ kleine Elektroden flächen ausgeglichen werden, welche vollständig ausreichend sind, da der Schwerpunkt und die Stärke dieser Behandlungsform in der lokalen Schmerztherapie liegt.

In der Praxis konnte festgestellt werden, daß die von den TNS-Geräten abgegebene Impulsform nicht den tatsächlichen Eigenschaften der TNS-Anwendung gerecht wird. Bisher werden die Stromstärkemessungen an einem rein ohmschen Lastwiderstand vorgenommen, wobei dieser Wert in den meisten Fällen etwa 1 KOhm beträgt. Die sich hieraus ergebende Impulsform entspricht einem Rechteckimpuls mit steilen Flanken.

Wie die Praxis nunmehr zeigt, besteht das Ersatzschaltbild nicht aus reinen ohmschen Lastwiderständen, sondern aus in Reihe liegenden Lastwiderständen mit einem parallel geschalteten Kondensator an einem Widerstand. Die sich hierdurch ergebenden Impulsformen weichen sehr stark von den zuvor genannten Impulsformen ab.

Es hat sich ferner gezeigt, daß die Dimensionierung der Bauelemente in dem vereinfachten Ersatzschaltbild von verschiedenen Parametern abhängt, nämlich von der Hautbeschaffenheit des Patienten, des verwendeten Elektrodengels, den Elektroden und der verwendeten Frequenz.

Es hat sich auch gezeigt, daß der gemessene Strom am Patienten größer als am 1 KOhm Meßwiderstand ist. Schließlich ist auch der am Patienten ermittelte Spannungsimpuls stark verschliffen, d.h. der Anstieg wird mit steilem Beginn flacher bis zu einem Maximum, welches steil abfällt und asymtotisch ausklingt. Der Stromimpuls verläuft sägezahnähnlich mit einem starken negativen Impuls.

Der Erfindung liegt die Aufgabe zugrunde, eine Schaltung anzugeben, welche Impulsformen erzeugt, die denen am Patienten gemessenen Impulsformen entspricht.

Die Lösung dieser Aufgabe besteht darin, daß ein durch den Ansteuerimpuls beaufschlagter Transistor T1 mit seinem Kollektor die Basis eines nachgeschalteten Transistors T2 ansteuert, derart, daß der Ansteuerimpuls im Transistor T1 invertiert wird, wobei der Transistor T2 nach der negativen Impulsflanke durchschaltet und der Ausgang über den emitterseitig verbundenen Entladewiderstand R(ent) kurzgeschlossen wird.

Nach der Erfindung ist die Basis des Transistors T1 der Ausgangskurzschlußschaltung über einen Eingangswiderstand R1 mit der Ansteuerung verbunden ist. Ferner ist der Kollektor des Transistors T2 mit dem einen Ausgangspol und der mit dem Emitter des Transistors T2 verknüpfte Entladewiderstand R(ent) mit dem anderen Ausgangspol verbunden.

Vorteilhaft ist es nach der Erfindung, wenn die Impulswiederholungsfrequenzen etwa maximal 160 Hz betragen.

Die Stromimpulsform ist sägezahnähnlich mit einer steilen negativen Abstiegsflanke ausgebildet, während die Spannungsimpulsform einen Anstieg mit steilem Beginn und flacher werdenden Verlauf bis zu einem Maximum aufweist, welche danach steil abfällt.

Das Ersatzschaltbild besteht aus in Reihe liegenden Lastwiderständen mit einem an einem Widerstand parallel geschalteten Kondensator.

Die Erfindung wird anhand der Zeichnungen näher beschrieben, hierbei zeigen:

FIGUR 1     ein Ersatzschaltbild der tatsächlichen Lasteigenschaften;

FIGUR 2     ein vereinfachtes Ersatzschaltbild nach Fig. 1;

FIGUR 3     Spannungs- und Stromimpulsformen, gemessen an 1 K Ohm;

FIGUR 4     Spannungs- und Stromimpulsformen, gemessen an einem Testpatienten;

FIGUR 5     Spannungs- und Stromimpulsformen, welche sich mit der Abschaltkurzschlußschaltung ergeben, und

FIGUR 6     die Abschaltkurzschlußschaltung.

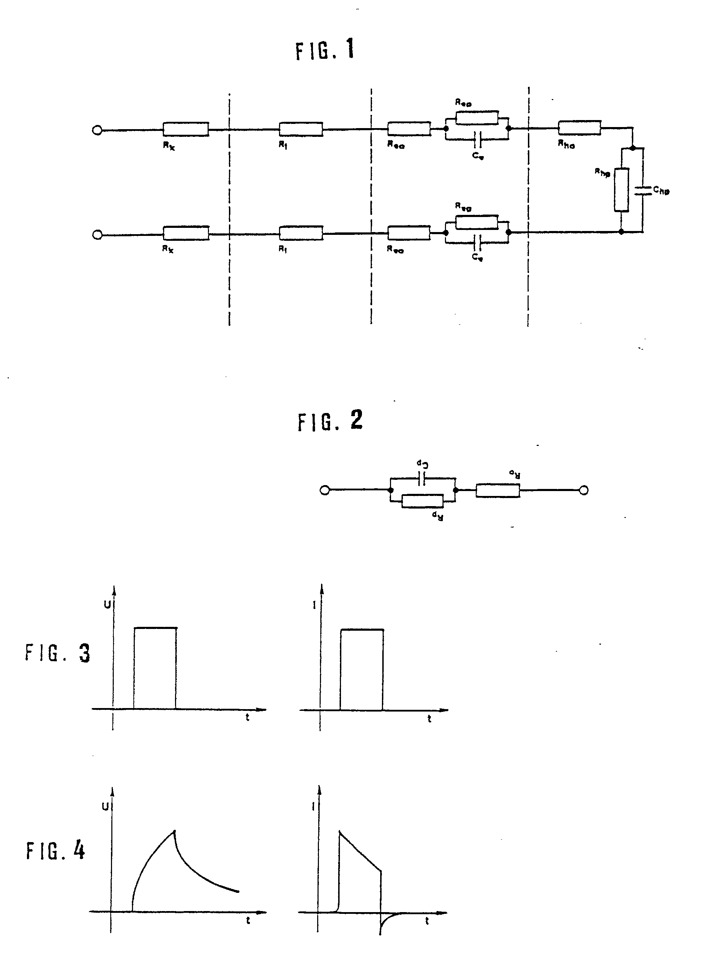

Die Figur 1 zeigt ein Ersatzschaltbild der tatsächlichen Lasteigenschaften. Diese besteht aus dem Kontaktwiderstand $R_k$, dem Leitungswiderstand $R_1$, dem Elektrodenwiderstand, dargestellt aus den in Reihe geschalteten Widerständen $R_e o$, $R_e p$ und dem zu $R_e p$ parallel geschalteten Kondensator $C_e$ und dem Hautwiderstand $R_h o$, $R_h p$ mit Parallel-Kondensator $C_h p$.

Die Figur 2 zeigt ein vereinfachtes Ersatzschaltbild, bestehend aus den in Reihe liegenden Widerständen $R_o$, $R_p$ und dem zu $R_p$ parallel liegenden Kondensator $C_p$.

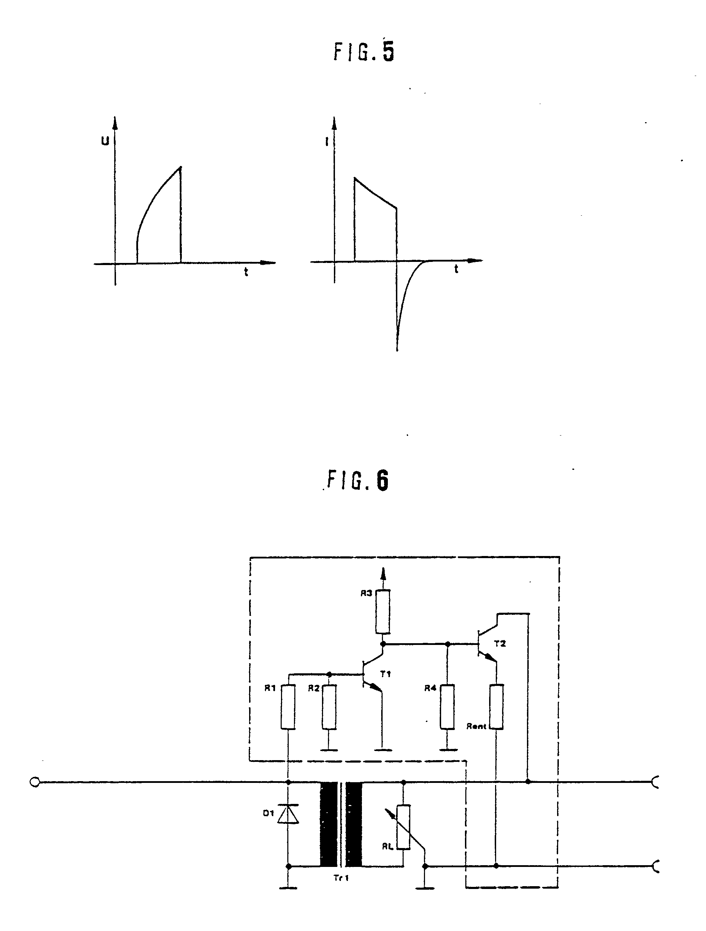

Die Figur 3 zeigt die Spannungs- und Stromimpulsform, gemessen an 1 KOhm und die Figur 4 im gleichen Maßstab die Spannungs- und Stromimpulsform, welche sich durch Messungen an Testpatienten ergibt. Der Vergleich der Figuren 3 und 4 zeigt die starke Abweichung. Es ist festzustellen, daß der Strom am Patienten etwa 10% bis 40% größer ist als am 1 KOhm Meßwiderstand.

Die Figur 5 zeigt die Spannungs- und Stromimpulsform, welche durch die Schaltung nach Figur 6 erreicht wird.

Die Ausgangskurzschlußschaltung nach Figur 6 weist einen Transistor T1 auf, der bei Beaufschlagung durch einen Ansteuerimpuls mit seinem Kollektor die Basis des nachgeschalteten Transistors T2 ansteuert. Hierbei wird der Ansteuerimpuls im Transistor T1 invertiert, wobei der Transistor T2 nach der negativen Impulsflanke (s. hierzu Figur 5) durchschaltet und der Ausgang über den emitterseitig verbundenen Entladewiderstand $R_e nt$ kurzgeschlossen wird. Hierdurch wird der Körperkondensator, der sich während des Impulses auflädt, sehr schnell entladen. Bei der nächsten positiven Ansteuerflanke sperrt T2 und der Impuls gelangt an den Patienten.

In der Therapie werden Impulswiederholungsfrequenzen vorteilhaft bis ca. 160 Hz verwendet. Bei höheren Frequenzen wird der körpereigene Kondensator durch den folgenden Impuls aufgeladen bevor er sich komplett entladen hat. Dies hat zur Folge, daß sich die Zellverbände mit steigender Frequenz nur noch sehr schwach erregen lassen.

Durch die Schaltung nach der Erfindung wird die Entladung des körpereigenen Kondensators stark beschleunigt.

Die Schaltung nach der Erfindung läßt sich vorteilhaft in allen bauartverwandten TNS-Geräten verwenden.

Die therapeutische Wirkung gegen Schmerzen wird dadurch erheblich verbessert.

**Patentansprüche**

1. Vorrichtung zur transkutanen elektrischen Nervenstimulation (TNS) mit einer Energieversorgung, einem Frequenzgenerator und einer elektrischen Schaltung zur Erzeugung unterschiedlicher Ausgangsfrequenzen veränderbarer Intensitäten an den mit der Vorrichtung verbindbaren Ausgangselektroden und einer Ausgangskurzschlußschaltung zur Verbesserung der abgegebenen Impulsform, **dadurch gekennzcichnet, daß** die Ausgangskurzschlußschaltung einen Transistor T1 aufweist, der bei Beaufschlagung durch einen Ansteuerimpuls mit seinem Kollektor die Basis eines nachgeschalteten Transistors T2 ansteuert, derart, daß der Ansteuerimpuls im Transistor T1 invertiert wird, wobei der Transistor T2 nach der negativen Impulsflanke durchschaltet und der Ausgang über den emitterseitig verbundenen Entladewiderstand R(ent) kurzgeschlossen wird.

2. Vorrichtung nach Anspruch 1, **dadurch gekennzeichnet, daß** die Basis des Transistors T1 der Ausgangskurzschlußschaltung über einen Eingangswiderstand R1 mit der Ansteuerung verbunden ist.

3. Vorrichtung nach Anspruch 1 oder 2, **dadurch gekennzeichnet, daß** der Kollektor des Transistors T2 mit dem einen Ausgangspol und der mit dem Emitter des Transistors T2 verknüpfte Entladewiderstand R(ent) mit dem anderen Ausgangspol verbunden ist.

4. Vorrichtung nach Anspruch 1, 2 oder 3, **dadurch gekennzeichnet, daß** die Impulswiederholungsfrequenzen etwa maximal 160 Hz betragen.

5. Vorrichtung nach Anspruch 1 oder einem der voranstehenden, **dadurch gekennzeichnet, daß** die Stromimpulsform sägezahnähnlich mit einer steilen negativen Abstiegsflanke ausgebildet ist.

6. Vorrichtung nach Anspruch 1 oder einem der voranstehenden, **dadurch gekennzeichnet, daß** die Spannungsimpulsform einen Anstieg mit steilem Beginn und flacher werdenden Verlauf bis zu einem Maximum aufweist, welche danach steil abfällt.

7. Vorrichtung nach Anspruch 1 oder einem der voranstehenden, **dadurch gekennzeichnet, daß** das Ersatzschaltbild aus in Reihe liegenden Lastwiderständen mit einem an einem Widerstand parallel geschalteten Kondensator besteht.

# FIG. 1

# FIG. 2

FIG. 3

FIG. 4

# FIG.5

# FIG.6

Europäisches
Patentamt

**EUROPÄISCHER
RECHERCHENBERICHT**

Nummer der Anmeldung

**EP 90 11 9844**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| Y | DE-U-8 910 361   (PIERENKEMPER) <br> * Figuren 1-2; Seite 3, Zeile 5 - Seite 6, Zeile 27 * <br> – – – | 1 | A 61 N 1/36 |
| Y | FR-E-6 170 6   (C.G.C.T.) <br> * Figuren 1-2; Seite 2, linke Spalte, Zeile 34 -rechte Spalte, Zeile 24 * | 1 | |
| A | | 2,3 | |
| | – – – | | |
| A | PATENT ABSTRACTS OF JAPAN, Band 11, Nr. 374 <br> (E-562)[2821], 5. Dezember 1987; <br> & JP-A-62 141 814 (MATSUSHITA) 25. Juni 1987 <br> – – – – – | 1-3 | |

|  |
|---|
| RECHERCHIERTE SACHGEBIETE (Int. Cl.5) |
| A 61 N <br> H 03 K |

Der vorliegende Recherchenbericht wurde für alle Patentansprüche erstellt

| Recherchenort | Abschlußdatum der Recherche | Prüfer |
|---|---|---|
| Den Haag | 04 Juni 91 | HERBELET J.C. |